# EUROPÄISCHE PATENTANMELDUNG

(11) **EP 0 599 007 A2**
(43) Veröffentlichungstag der Anmeldung: **01.06.1994**
(21) Anmeldenummer: 93114956.1
(22) Anmeldetag: 16.09.1993
(51) Int. Cl.: A61B 17/32, A61B 17/22

(54) **Verfahren und Vorrichtung zum selektiven Schneiden von biologischem Gewebe**

(30) Priorität: 04.11.1992 DE 4237154
(71) Anmelder: DORNIER MEDIZINTECHNIK GMBH, D-82101 Germering (DE)
(72) Erfinder: Rother, Werner, Dr., D-64331 Weilerstadt (DE); Hauptmann, Gerhard, D-81827 München (DE); Frank, Frank, Dr., D-85560 Ebersberg (DE); Hessel, Stefan, Dr., D-81927 München (DE)
(74) Vertreter: Frick, Gerhard, Dipl.-Ing.

(57) **Zusammenfassung**

Verfahren und Vorrichtung zum selektiven Schneiden von biologischem Gewebe, wobei Lichtimpulse eines Pulslaser über eine Lichtleitfaser (4) in eine zumindest teilweise mit einer Flüssigkeit gefüllten Röhre (3) geleitet werden, dort aus der Lichtleitfaser (4) austreten und innerhalb der Röhre (3) in der Flüssigkeit vollständig absorbiert werden, so daß aufgrund mindestens eines absorbierten Lichtimpulses in der Röhre (3) eine Flüssigkeitssäule in Richtung auf das zu schneidende Gewebe beschleunigt wird.

## Beschreibung

Die Erfindung betrifft ein Verfahren sowie eine Vorrichtung zum selektiven Schneiden von biologischem Gewebe mit einem pulsierenden Flüssigkeitsstrahl.

Eine Reihe von chirurgischen Eingriffen erfordert ein separates Präparieren von biologischem Gewebe und den darin eingebetten Gefäßsystemen. Dies ist unter Verwendung von konventionellen chirurgischen Instrumenten oft sehr schwierig und in manchen Fällen nur sehr unvollständig durchführbar. Infolgedessen werden oftmals wichtige Gefäße versehentlich perforiert oder durchtrennt. Einen besonders kritischen Fall stellt die Leberresektion dar, bei es häufig zu hohen Blutverlusten kommt, die durch entsprechende Bluttransfusionen ausgeglichen werden müssen.

Zur Durchtrennung weicherer Gewebepartien unter Schonung der im allgemeinen festeren Gefäße ist eine Wasserstrahlmethode bekannt, bei der Wasser unter sehr hohem Druck durch sehr feine Düsen gepreßt wird und somit Parenchymgewebe selektiv und berührungsfrei geschnitten werden kann. Die medizinische Anwendung eines solchen Jet-Cutters wird u.a. in Rau H.G., Arnold H., Schildberg F.W., (1990) Schneiden mit dem Wasser-Strahl (Jet-Cutting) - eine Alternative zum Ultraschallaspirator? Chirurg (1990) 61: 735-738 oder in der EP 0 489 496 A1 näher beschrieben. Der Nachteil einer derartigen Wasserstrahlmethode besteht darin, daß der Druckimpuls am proximalen Ende der Leitung erzeugt wird und dadurch bis zum Auftreffen auf das Gewebe. an Steilheit und Amplitute verliert. Die mechanische Ausführung solcher Geräte erfordert druckfeste Schläuche, die steif und unhandlich sind. Die Handstücke mit sehr feinen Düsen müssen ebenfalls druckfest ausgelegt werden, dadurch ergibt sich ein schwerer und unhandlicher Aufbau. Die feinen Düsen können sehr leicht zu Verstopfungen führen.

Aus der EP 0 454 312 A2 ist ein Laserlithotripter bekannt, bei dem insbesondere zur Fragmentierung schlecht absorbierender Konkremente eine Stoßwelle in der Spülflüssigkeit zwischen einer das Laserlicht führenden Lichtleitfaser und dem Konkrement erzeugt wird. Die Stoßwelle soll dann die Fragmentierung des Konkrementes bewirken, wobei das Ende der Lichtleitfaser so nahe an das Konkrement herangeführt werden muß, daß einerseits noch die Absorption der Laserstrahlung in der Flüssigkeit und nicht im Konkrement erfolgt, andererseits jedoch noch keine nennenswerte Dämpfung der Stoßwelle stattfindet.

Es ist Aufgabe der vorliegenden Erfindung, ein Verfahren sowie eine leicht handhabbare Vorrichtung zu schaffen, mit der biologisches Gewebe selektiv geschnitten, abgetragen oder fragmentiert werden kann. Zusätzlich sollen auch vom Material unabhängige Schnitte durchgeführt und Gewebe koaguliert werden können.

Diese Aufgabe erfüllt ein Verfahren mit den Merkmalen des Patentanspruchs 1 bzw. eine Vorrichtung nach den Merkmalen des Patentanspruchs 4.

Mit dem erfindungsgemäßen Verfahren bzw. der Vorrichtung werden besonders feine und gut dosierbare Flüssigkeitsimpulse erzeugt, die ein besonders feines und selektives Schneiden bzw. Abtragen oder Fragmentieren von biologischem Gewebe ermöglicht. Dies wird dadurch erreicht, daß Laserimpulse, welche in einer Flüssigkeit möglichst vollständig absorbiert werden, Stoßwellen erzeugen, die wiederum die Flüssigkeit in Richtung auf das zu behandelnde biologische Gewebe beschleunigen und dort nach Art eines pulsierenden Wasserstrahles Gewebe bis zu einer bestimmten Festigkeit schneiden, abtragen oder fragmentieren und somit festeres Gewebe, wie z.B. Blutgefäße, Knorpelmasse u. dgl. unbeschädigt lassen. Die Flüssigkeitsimpulse können somit in unmittelbarer Nähe des zu behandelnden Gewebes erzeugt werden, so daß sich deren Pulsform bis zum Auftreffen auf das Gewebe kaum mehr verändert. Sollen auch solche festeren Gewebeteile durchtrennt werden, so kann die Laserstrahlung in bekannter Weise direkt auf das Gewebe appliziert werden. Dies kann entweder dadurch geschehen, daß die Flüssigkeit, in der Regel eine Spülflüssigkeit, zwischen dem distalen Ende der Lichtleitfaser und dem Gewebe beseitigt wird oder, daß die Lichtleitfaser direkt zum Zwecke des Kontaktschneidens auf das Gewebe aufgesetzt wird. Bei frei applizierter Laserstrahlung ist selbstverständlich auch eine Koagulation von Gewebe möglich.

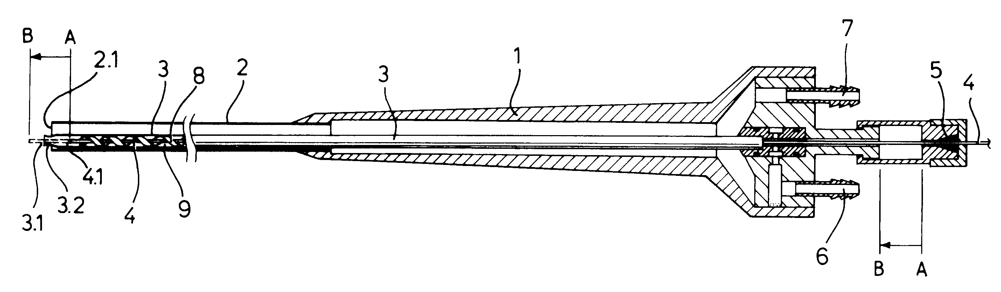

Die Erfindung wird im folgenden anhand eines in der Figur teilweise schematisch dargestellten Ausführungsbeispieles näher beschrieben.

Die Figur zeigt einen Querschnitt durch ein Handstück zum selektiven Schneiden von biologischem Gewebe. Das Handstück weist einen hohlen bleistiftförmigen Griff 1 auf, dessen distales Ende in einem ovalen Rohr 2 (im weiteren Mantelrohr genannt) mündet. Im Inneren des Griffes 1 sowie des Mantelrohres 2 verläuft ein zweites zylindrisches Rohr 3, dessen Ende 3.1 etwas aus dem Mantelrohr 2 hinausragt. Das Rohr 3 dient als Führungsrohr für eine Lichtleitfaser 4, welche im hinteren Teil des Griffstückes durch eine verschiebbare Klemmvorrichtung 5 fixiert ist und zwischen den beiden Positionen A und B axial verschiebbar ist. Im hinteren Teil des Handstückes sind zwei Anschlüsse 6 und 7 vorgesehen, von denen der Anschluß 6 mit einer Spülflüssigkeitsquelle und der Anschluß 7 mit einer Absaugeinrichtung verbunden ist. Die Spülflüssigkalt strömt durch den Anschluß 6 in einen Kanal zwischen der Lichtleitfaser 4 und dem Führungsrohr 3 zum distalen Ende 3.1. Dort wird die Flüssigkeit durch einen Kanal zwischen dem Führungsrohr 3 und dem Mantelrohr 2 bzw. dem Griff 1 über den Anschluß 7 wieder abgesaugt.

Die Lichtleitfaser 4 ist mit einem nicht dargestellten Holmium: YAG-Laser verbunden, der eine Impulsenergie zwischen 500 - 3000 m J mit einer Impulsdauer zwischen 150 und 250 µsec und einer Pulsfrequenz zwischen 3 - 20 Hz abgeben kann.

Zum selektiven Schneiden wird die Lichtleitfaser in die Position A geschoben, wobei deren distales Ende 4.1 sich im Inneren des Führungsrohres 3 einige Millimeter vor dessen distalem Ende 3.1 befindet. Das Führungsrohr 3 wird dann auf das zu schneidende Gewebe aufgesetzt und die Spülflüssigkeitszufuhr sowie die Absaugeinrichtung in Gang gesetzt. Daraufhin wird der Holmium:YAG-Laser in Betrieb genommen, dessen Laserimpulse aufgrund ihrer Wellenlänge von ca. 2,1 µm in der Spülflüssigkeit zwischen dem distalen Ende 4.1 der Lichtleitfaser 4 und dem distalen Ende 3.1 des Führungsrohres 3 vollständig absorbiert werden. Jeder Impuls löst eine Stoßwelle aus, die die vor dem Ende der Lichtleitfaser befindliche Flüssigkeitssäule aus dem Führungsrohr 3 herausbeschleunigt. Dieser pulsartige Flüssigkeitsstrahl hat die Eigenschaft, daß er weiches Gewebe durchtrennt, abträgt oder fragmentiert, jedoch härtere Bestandteile, wie z.B. Blutgefäße, unbeschädigt läßt. Die abgetrennten bzw. fragmentierten Gewebestücke werden durch das Mantelrohr 2 zusammen mit der Spülflüssigkeit sofort abgesaugt.

Die Breite des Schnittes, die insbesondere zum Freilegen von Blutgefäßen ein gewisses Maß nicht unterschreiten sollte, wird wesentlich durch die Austrittsöffnung 3.1 des Führungsrohres 3 bestimmt. Diese kann z.B. als Düse geformt sein, wobei der kleinste Düsenquerschnitt etwas größer als der äußere Durchmesser der Lichtleitfaser 4 ist.

Zum Zentrieren der Lichtleitfaser 4 in dem Führungsrohr 3 sowie zur rückwärtigen Dämmung gegen die ausgelösten Schockwellen und somit zur Effizienzsteigerung der abgegebenen Flüssigkeitsimpulse ist zwischen der Lichtleitfaser 4 und dem Führungsrohr 3 ein Kunststoffband 8 schraubenförmig um die Lichtleitfaser 4 gewickelt derart, daß zwischen den einzelnen Gewindegängen ein schraubenförmiger Kanal 9 für die Spülflüssigkeit freibleibt.

Zum feinen, nicht selektiven Schneiden bzw. zum Koagulieren, wird die Lichtleitfaser von der Position A in die Position B geschoben, so daß sie aus dem Führungsrohr 3 herausragt. In dieser Position arbeitet das Gerät wie ein an sich bekanntes chirurgischen Laserinstrument.

Treten beim Schneiden kleine lokale Blutungen auf, so können diese ebenfalls mit dem Instrument koaguliert werden. Dazu wird die Spülung abgestellt, die Lichtleitfaser 4 in die Position B geschoben und dann das Gewebe berührungsfrei mit der Laserstrahlung behandelt.

Die Stärke der Flüssigkeitspulse läßt sich zum einen durch eine Leistungsregelung des Laser zum anderen jedoch auch durch Einstellung des Abstandes zwischen dem distalen Ende 4.1 der Lichtleitfaser 4 und dem Ende 3.1 des Führungsrohres 3 einstellen.

Anstelle eines Holmium:YAG-Lasers können selbstverständlich auch andere Laser mit einem Wellenlängenbereich zwischen 1,4 und 4,5 µm zur Anwendung kommen, z.B. ein Erbium:YAG-Laser mit einer Wellenlänge von 2,9 µm.

## Patentansprüche

1. Verfahren zum selektiven Schneiden von biologischem Gewebe mit einem pulsierenden Flüssigkeitsstrahl **dadurch gekennzeichnet**, daß Lichtimpulse eines Pulslasers über eine Lichtleitfaser in eine zumindest teilweise mit einer Flüssigkeit gefüllten Röhre geleitet werden, dort aus der Lichtleitfaser austreten und innerhalb der Röhre in der Flüssigkeit vollständig absorbiert werden und, daß aufgrund mindestens eines absorbierten Lichtimpulses in der Röhre eine Flüssigkeitssäule in Richtung auf das zu schneidende Gewebe beschleunigt wird.

2. Verfahren nach Anspruch 1, **dadurch gekennzeichnet**, daß während des selektiven Schneidvorgangs durch die Röhre eine Spülflüssigkeit in distaler Richtung strömt, auf das zu schneidende Gewebe auftrifft und zusammen mit beim Schneiden entstehenden Gewebepartikeln durch einen koaxial oder parallel zur Röhre verlaufenden Kanal abgesaugt wird.

3. Verfahren zum wechselweisen selektiven Schneiden mit einem pulsierenden Flüssigkeitsstrahl oder Kontaktschneiden von biologischem Gewebe nach Patentanspruch 1 oder 2, **dadurch gekennzeichnet**, daß das distale Ende der Lichtleitfaser von einer ersten, innerhalb einer zumindest teilweise mit einer Flüssigkeit gefüllten Röhre befindlichen Position zu einer zweiten, außerhalb der Röhre befindlichen Position verschoben wird oder umgekehrt.

4. Vorrichtung zum selektiven Schneiden von biologischem Gewebe mit einem pulsierenden Flüssigkeitsstrahl , **gekennzeichnet** durch eine mit einem Pulslaser verbundene Lichtleitfaser (4), deren distales Ende (4.1) innerhalb einer dünnen mit einem Ende auf das Gewebe richtbaren Röhre (3) angeordnet und dort von einer Flüssigkeit umgeben ist, wobei die aus der Lichtleitfaser (4) austretenden Laserimpulse innerhalb der Röhre (3) in der Flüssigkeit absorbierbar sind.

5. Vorrichtung nach Anspruch 4, **dadurch gekennzeichnet**, daß die Röhre (3) mit ihrem proximalen Ende an eine Spülflüssigkeitsquelle angeschlossen ist.

6. Vorrichtung nach Anspruch 4 oder 5, **dadurch gekennzeichnet,** daß die Röhre (3) von einem Mantelrohr (2) umgeben ist, welches mit einer Absaugeinrichtung verbunden ist.

7. Vorrichtung nach einem der Ansprüche 4 oder 6, **dadurch gekennzeichnet,** daß die Lichtleitfaser (4) in der Röhre (3) axial verschiebbar ist.

8. Vorrichtung nach Anspruch 6 oder 7, **dadurch gekennzeichnet,** daß das distale Ende (3.1) der Röhre (3) aus dem distalen Ende (2.1) des Mantelrohres (2) herausragt und das distale Ende (4.1) der Lichtleitfaser (4) aus der Röhre (3) herausschiebbar ist.

9. Vorrichtung nach einem der Ansprüche 4 bis 8, **dadurch gekennzeichnet**, daß in der Röhre (3) ein flüssigkeitsdurchlässiger, schockwellendämmender Körper angeordnet ist.

10. Vorrichtung nach Anspruch 9, **dadurch gekennzeichnet**, daß die Lichtleitfaser (4) innerhalb der Röhre (3) von einem Band (8) schraubenförmig umgeben ist.

11. Vorrichtung nach einem der Ansprüche 4 bis 10, **dadurch gekennzeichnet**, daß die Wellenlänge des Pulslasers derart ausgewählt oder eingestellt wird, daß dessen Strahlung in Wasser einen Absorptionskoeffizienten α > 10cm⁻¹ erreicht.

12. Vorrichtung nach Anspruch 11, **dadurch gekennzeichnet**, daß als Pulslaser ein Erbium: YAG oder ein Holmium:YAG-Laser verwendet wird.
